# EUROPEAN PATENT SPECIFICATION

(11) **EP 2 433 674 B1**
(45) Date of publication and mention of the grant of the patent: **05.06.2013**
(21) Application number: 10178993.1
(22) Date of filing: 23.09.2010
(51) Int. Cl.: A61N 1/362, A61N 1/365, A61N 1/368, A61B 5/0215, A61B 5/00, A61N 1/372

(54) **Systems for stimulating a heart**
Systeme für Herzstimulation
Systèmes de stimulation cardiaque

(43) Date of publication of application: 28.03.2012
(73) Proprietor: St. Jude Medical AB, 175 84 Järfälla (SE)
(72) Inventor: Blomqvist, Andreas, SE-187 76, Täby (SE); Persson, Torbjörn, SE-211 12, Malmö (SE); Hill, Rolf, SE-175 55, Järfälla (SE)
(74) Representative: Sjölander, Henrik

(56) References cited:
- US-A1- 2002 151 938
- US-A1- 2008 103 539
- US-A1- 2009 292 334

## Description

### Field of the invention

The present invention relates generally to implantable medical devices and more particularly to systems for stimulating a heart of a patient.

### Background of the invention

Heart failure is usually a chronic, long term condition, but may occur suddenly. It may affect the left heart, the right heart, or both sides of the heart. Heart failure may be considered as a cumulative consequence of all injuries and/or stress to the heart over a person's life and the prevalence of heart failure increases constantly. For example, it is estimated that nearly 5 million people in the USA suffer from heart failure and about 400.000 new cases are diagnosed every year. The prevalence of heart failure approximately doubles with each decade of life. One of the most important means of treating heart failure is cardiac resynchronization therapy, CRT. Although CRT is a very effective way of treating heart failure in most patients there is a large percentage for which the CRT has no apparent effect at all. Different estimates of the size of the so called group "non-responders" exist, but it is generally believed to be in the vicinity of 25 % of all patients equipped with a CRT device. However, there are numbers reported to be as high as 33 % (depending mostly on the definition of CRT response which may vary greatly).

A common method for adapting the therapy (the timing cycles) for non-responders is so called echo-based optimization, which may include M-mode, 2D, 3D and TDI. Echo-based optimization of the timing cycles is often time-consuming and may range from 30 minutes to two hours depending on the scope of the evaluation. Furthermore, echo-based optimization is heavily dependent of the operator, who interprets the displayed echo signals, for accuracy and consistency. Accordingly, there is a need for more reliable, fast, and accurate methods for CRT timing optimization and for patient customized CRT timing optimization.

Device based CRT optimization is likely to be one of the most potent tools in improving CRT efficiency and more specifically fighting non-responders. St. Jude Medical's QuickOpt^{™} Timing Cycle Optimization is an algorithm that provides IEGM (Intracardiac Electrogram) based AV (Atrial-Ventricular) timing optimization in CRT and ICD (Implantable Cardioverter-Defibrillator) systems and VV (Ventricular-Ventricular) timing optimization in CRT devices in a simple and swift way. QuickOpt^{™} Timing Cycle Optimization is based on the hypothesis that the point of time for the closure of the Mitral valve can be estimated by measuring the interatrial conduction time (P-wave duration), that the onset of isovolumetric contraction can be measured using the peak of the R-wave and that interventricular conduction delays can be measured by evaluating simultaneous RV (Right Ventricular) and LV (Left Ventricular) IEGMs and measuring the time between the peaks of the R-waves. The goal is to characterize interatrial conduction patterns so that preload is maximized and ventricular pacing does not occur until after full closure of the mitral valve and to characterize intrinsic and paced interventricular conduction patterns so that pacing stimuli and the resultant LV and RV conduction (paced wave fronts) meet at the ventricular septum. Accordingly, QuickOpt^{™} Timing Cycle Optimization electrically characterizes the conduction properties of the heart to calculate optimal AV delay, PV delay (the time interval between a sensed atrial event and the ventricular impulse) and VV delay. QuickOpt^{™} Timing Cycle Optimization has been clinically proven to correlate with the more time-consuming echo-based methods and may be used for patients carrying CRT and dual-chamber devices at implant or follow up. QuickOpt^{™} Timing Cycle Optimization is an appealing optimization method since it does not require systematic measurements of a number of different AV and VV delays, which makes it very fast and simple. There are other IEGM based optimization methods among which QuickOpt^{™} Timing Cycle Optimization is one such method.

Despite the evident advantages of IEGM based optimization methods, such as e.g. QuickOpt^{™} Timing Cycle Optimization, there is an opinion within the medical community, for example, among physicians that results, e.g. timing cycles, based on input data more directly reflecting the mechanical functioning of the heart may be even more accurate and reliable. Thus, there is still a need within the art for further improved methods and devices for optimizing AV and VV delays.

US 2002/151938 A1, which discloses all the features of the preamble of claim 1, discloses a medical device system for optimizing AV and VV delays based on a myocardial performance index calculated from electrical (QT interval) and mechanical (timing of first and second heart sounds) parameters. First, a range of AV delays is applied and the corresponding performance indexes are calculated and stored. When the end of the range has been reached, the optimal performance index value is identified (e.g. as being the maximum value or the minimum value) and therapy configured to the corresponding AV delay. Then, in a similar fashion, a range of VV delays is applied, the optimal performance index value identified, and therapy configured to the corresponding VV delay.

### Summary of the invention

An object of the present invention is to provide a system for further improvements in optimization of AV and VV delays for use in, for example, CRT.

This and other objects of the present invention are achieved by means of an implantable medical device having the features defined in the independent claim. Embodiments of the invention are characterized by the dependent claims.

According to an aspect of the present invention, there is provided a system comprising a cardiac stimulator for stimulating a heart of a patient. The cardiac stimulator is connectable to a hemodynamic sensor adapted to sense hemodynamic signals. The system further comprises a pacing module arranged in the cardiac stimulator adapted to provide pacing signals for delivery to the patient via medical leads including electrodes connectable to the cardiac stimulator, a control module arranged in the cardiac stimulator adapted to control settings of the pacing module to deliver pacing signals in accordance with preset AV and VV delays, a data collection module arranged in the cardiac stimulator adapted to collect hemodynamic signals from the hemodynamic sensor during preset AV delays and VV delays, a data processing module arranged in the cardiac stimulator and being coupled to the data collection module, and an optimization module adapted to optimize an AV and VV delay based on IEGM signals obtained via the medical leads to determine recommended AV and VV delays.

The data processing module is adapted to: determine a delay control parameter for each preset AV delay and VV delay based on the collected hemodynamic signals for respective preset AV delay and VV delay; evaluate the settings for the preset AV delays and VV delays to determine the AV delay setting that corresponds to the maximum delay control parameter and the VV delay setting that corresponds to the maximum delay control parameter; determine an AV delay error correction value as a difference between the AV delay corresponding to the maximum delay control parameter and a recommended AV delay; and determine a VV delay error correction value as a difference between the VV delay corresponding to the maximum delay control parameter and a recommended VV delay.

The present invention is based on the insight that an IEGM based optimization of timing cycles, such as QuickOpt^{™} Timing Cycle Optimization, can be further improved by using error correction values created by means of hemodynamic signals reflecting the mechanical functioning of the heart. QuickOpt^{™} Timing Cycle Optimization is based on the hypothesis that the point of time for mitral valve closure can be estimated by measuring the interatrial conduction time (P-wave duration), that onset of isovolumetric contraction can be measured using the peak of the R-wave, and that interventricular conduction delays can be measured by evaluating simultaneous RV and LV IEGMs and measuring the time between peaks of the R-waves.

Since the AV and VV delays calculated using IEGM based optimization such as QuickOpt^{™} Timing Cycle Optimization are partly based on a hypothetical foundation, the inventors have realized that the AV and VV delays can be made more accurate by using error correction parameters based on hemodynamic signals, which directly reflect the mechanical functioning of the heart.

In fact, the optimization can be improved in a number of different ways by including hemodynamic data and modifying the AV and VV delays based on parameters calculated using this hemodynamic data.

For example, studies have shown that AV and VV delays based on hemodynamic signals may be more reliable and accurate than IEGM based delays. For example, there are studies showing a poor correlation between IEGM based delays and delays based on pressure measurements, e.g. LV dP/dtₘₐₓ, ("The Optimized V-V interval Determined by Interventricular Conduction Times versus Invasive Measurement by LV dP/dtmax", van Gelder, et al.). Hence, the reliability and accuracy of the AV and VV delays, and thus the CRT, can be improved using the combined IEGM and hemodynamically based optimization as suggested by the present invention. Furthermore, the IEGM based optimizations, such as e.g. QuickOpt^{™} Timing Cycle Optimization, may not deliver the optimal AV and VV delays for all patients due to the fact that they are to some extent based on a hypothesis created from average values and parameters collected from a large number of patients. Thereby, there might be individual patients for whom the IEGM based AV and VV delays are not suitable. By adapting and tailoring the IEGM based delays with data from the hemodynamic measurements obtained from the specific patient after implantation, better AV and VV delays adapted for that patient can be created.

Further, the invention has also been developed taking into consideration the problems associated with hemodynamic sensors in a chronic setting. An optimization of AV and VV delays based only on hemodynamic data may not be reliable due to problems related to, for example, overgrowth and clogging, which may lead to a drift of the sensor signals delivered.

Moreover, hemodynamic sensors are often also sensitive to the physical placement. An erroneous or less optimal placement of a sensor (or lead) may lead to a perturbed signal.

Further, hemodynamic sensors are often sensitive to motion artifacts and body-noise.

In addition, an optimization based only on hemodynamic data would probably lead to high power consumption since the hemodynamic sensor generally has relatively high power consumption.

In light of this, the present invention is also based on the idea of determining AV and VV delay, for example, for CRT by combining the IEGM based optimization with results based on hemodynamic data, and by modifying the AV and VV delays from the IEGM based optimization with correction values obtained based on hemodynamic data.

Thus, the present invention makes use of the advantages of the IEGM based optimization and hemodynamically based sensor data, respectively. This is achieved in that the IEGM based optimization is used to determine rough initial or start values for the AV and VV delay, which can be done in a prompt and quick way. The more time consuming and power consuming hemodynamic data collection is then performed to create correction values to the initially determined AV and VV delays. The correction values can also be iteratively improved over time.

According to embodiments of the present invention, the recommended AV and VV delay are updated with the AV and VV delay error correction value, respectively, to obtain a modified recommended AV and VV delay. These modified AV and VV delays may be further redefined in an iterative process, i.e. by iteratively modifying AV and VV delay error correction values with new AV and VV delay error correction values obtained in successive procedures. That is, current AV and VV delay error correction values are modified using at least one set of AV and VV delay error correction values obtained from a subsequent determination of AV and VV delay error correction values.

According to an embodiment of the present invention, the pacing module is controlled to deliver pacing signals in accordance with preset VV delays at a specific AV delay, the specific AV delay being the AV delay corresponding to the maximum delay control parameter. Moreover, a delay control parameter for each VV delay is determined based on the collected hemodynamic signals for respective VV delay obtained at the specific AV delay; and the settings for the VV delays are evaluated to determine the VV delay setting that corresponds to the maximum delay control parameter at the specific AV delay. This provides a quicker and more efficient algorithm in terms of calculation time and battery consumption since a fewer number of VV delay measurements have to be performed and hence a fewer number of delay control parameters have to be determined.

According to embodiments of the present invention, the hemodynamic sensor is an implantable pressure sensor. In specific embodiments, the pressure sensor is adapted for transmural placement so as to enable sensing of a left ventricular pressure. A suitable pressure sensor is, for example described, in the co-pending application PCT/EP2010/058624 by the same applicant, and in US RE 39,863, US 6,248,083, or US RE 35,648. If the pressure sensor as described in the co-pending application PCT/EP2010/058624 is used, it is possible to implant the pressure sensor without any punching/drilling of hole in the septum and it is possible to implant the pressure sensor transeptally from the right ventricle to the left ventricle. The pressure sensor can be placed fast and without any additional tools required than what is normally used for lead implantation. The sensor can easily be pushed through the myocardium for access to the left side with minimal damage to the tissue of the septum. This pressure sensor however has a limited (less than a year) useful life due to, for example, overgrowth.

According to embodiments of the present invention, the hemodynamic sensor is selected from the group including: accelerometers, blood flow probes, load indicators which react to geometrical changes, heart sound sensors, and photoplethysmography sensors.

In embodiments of the present invention, the cardiac stimulator further comprises a communication module adapted to communicate with extracorporeal equipment wirelessly using RF or inductive telemetry.

According to embodiments of the present invention, the optimization module is arranged in extracorporeal equipment having a communication module for communication with the communication module of the cardiac stimulator.

In other embodiments of the present invention, the optimization module is arranged in the cardiac stimulator.

According to embodiments of the present invention, the left ventricular pressure derivative is calculated for each AV and VV delay, wherein the delay control parameter is the left ventricular pressure derivative.

According to an embodiment of the present invention, a specific heart rate interval based on the sensed heart rate at which the hemodynamic signals were obtained is determined. Thereby, according to some embodiments, an approximate heart rate or heart rate interval is associated with or labeled to each AV or VV error correction parameter. The AV and VV delay error correction values may be binned together according to what approximate heart rate or heart rate interval at which they were acquired. For example, if a first procedure to obtain AV and VV delay error correction values were performed between 65 - 75 bpm, and a second procedure to obtain AV and VV delay error correction values were performed between 75 - 85 bpm, the results from these procedures will not be mixed. Instead, this will result in two sets of error correction values, one set of AV and VV delay error correction values of the heart rate interval between 65 - 75 bpm and one set of AV and VV delay error correction values of the heart rate interval between 75 - 85 bpm. Thus, it is possible to obtain heart rate specific error correction values and, in turn, heart rate specific AV and VV delays. Thereby, it is possible to obtain rate dependent error correction terms and AV and VV delays.

Further objects and advantages of the present invention will be discussed below by means of exemplifying embodiments.

These and other features, aspects and advantages of the invention will be more fully understood when considered with respect to the following detailed description, appended claims and accompanying drawings.

### Brief description of the drawings

Exemplifying embodiments of the invention are illustrated by way of example and not by way of limitation in the figures of the accompanying drawings in which like references indicate similar elements. It should be noted that references to "an" or "one" embodiment in this discussion are not necessarily to the same embodiment, and such references mean at least one.
Fig. 1 is a simplified diagram of one embodiment of an implantable stimulation device in electrical communication with several leads implanted in a patient's heart for measuring hemodynamic signals delivering multi-chamber stimulation.
Fig. 2 is a simplified functional block diagram of one embodiment of a system in accordance with the present invention, illustrating basic elements of the system.
Fig. 3 is a simplified functional block diagram of another embodiment of a system in accordance with the present invention, illustrating basic elements of the system.
Fig 4 is a flow chart illustrating general steps of a method for optimizing AV and VV delays.
Fig. 5 is a flow chart illustrating steps of an illustrative example of the method for optimizing AV and VV delays.

### Description of exemplifying embodiments

The following is a description of exemplifying embodiments in accordance with the present invention. This description is not to be taken in limiting sense, but is made merely for the purposes of describing the general principles of the invention. It is to be understood that other embodiments may be utilized and structural and logical changes may be made without departing from the scope of the present invention. Some embodiments of the present invention relate generally to implantable pressure sensors but, however, even though particular types of pressure sensors are mentioned herein, the present invention is not limited to pressure sensors but may include other types of hemodynamic sensors such as, accelerometers, blood flow probe, load indicators which react to geometrical changes, heart sound sensors, or photoplethysmography sensors or similar sensors.

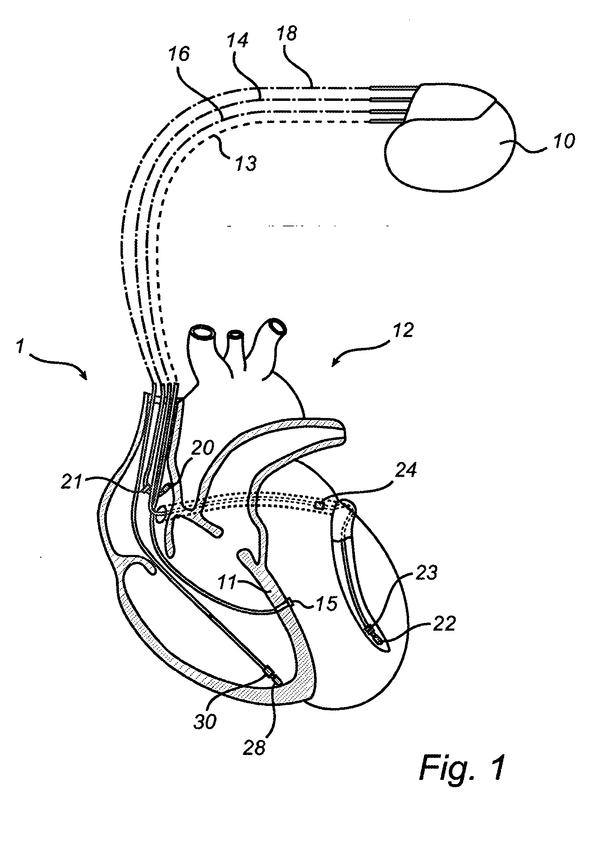

Referring to Fig. 1, one implementation of the present invention relating to a system including an implantable cardiac stimulator connectable to one or more medical leads will be discussed. In this embodiment the cardiac stimulator is described as being connectable to an implantable pressure sensor but, as mentioned above, there are a number of other hemodynamic sensors that may be conceivable.

The implantable cardiac stimulator 10 of the system 1 is in electrical communication with a patient's heart 12 by way of three leads 14, 16, and 18 suitable for delivering multichamber stimulation therapy. Further, the cardiac stimulator 10 is connected to a hemodynamic sensor 15, which in one particular embodiment of the present invention is a pressure sensor 15 attached to septum 11 arranged in a medical lead 13 coupled to the cardiac stimulator 10. A suitable pressure sensor is, for example described, in the co-pending application PCT/EP2010/058624 by the same applicant, US RE 39,863, US 6,248,083, or US RE 35,648. However, as mentioned above, other types of hemodynamic sensors may alternatively or as a complement be used including accelerometers for measuring pressure changes in the left ventricle, flow probes, load indicators for measuring geometrical changes in the cardiac tissue e.g. in septum, heart sound sensors, or photoplethysmographic sensors.

To sense atrial signals and to provide right atrial chamber stimulation therapy, the stimulator 10 is coupled to an implantable right atrial lead 14 having, for example, an atrial tip electrode 20, which typically is implanted in the patient's right atrial appendage or septum. Fig. 1 shows the right atrial lead 14 as also having an atrial ring electrode 21.

To sense left atrial and ventricular cardiac signals and to provide left chamber pacing therapy the stimulator is coupled to a coronary sinus lead 16 designed for placement in the coronary sinus region via the coronary sinus for positioning a distal electrode adjacent to the left ventricle and/or additional electrod(s) adjacent to the left atrium. As used herein, the phrase "coronary sinus region" refers to the vasculature of the left ventricle, including any portion of the coronary sinus, great cardiac vein, left marginal vein, left posterior ventricular vein, middle cardiac vein, and/or small cardiac vein or any other cardiac vein accessible via the coronary sinus.

The lead 16 is designed to receive atrial and ventricular cardiac signals and to deliver left ventricular pacing therapy using, for example, a left ventricular tip electrode 22, a left ventricular ring electrode 23, and left atrial pacing therapy using, for example, a left atrial ring electrode 24.

The cardiac stimulator 10 is also in electrical communication with the heart 12 by way of an implantable right ventricular lead 18 having, in this embodiment, a right ventricular tip electrode 28 and a right ventricular ring electrode 30. Typically, the right ventricular lead 18 is transvenously inserted into the heart 12 to place the right ventricular tip electrode 28 in the right ventricular apex. The right ventricular lead 18 is capable of sensing or receiving cardiac signals, and delivering stimulation in the form of pacing therapy.

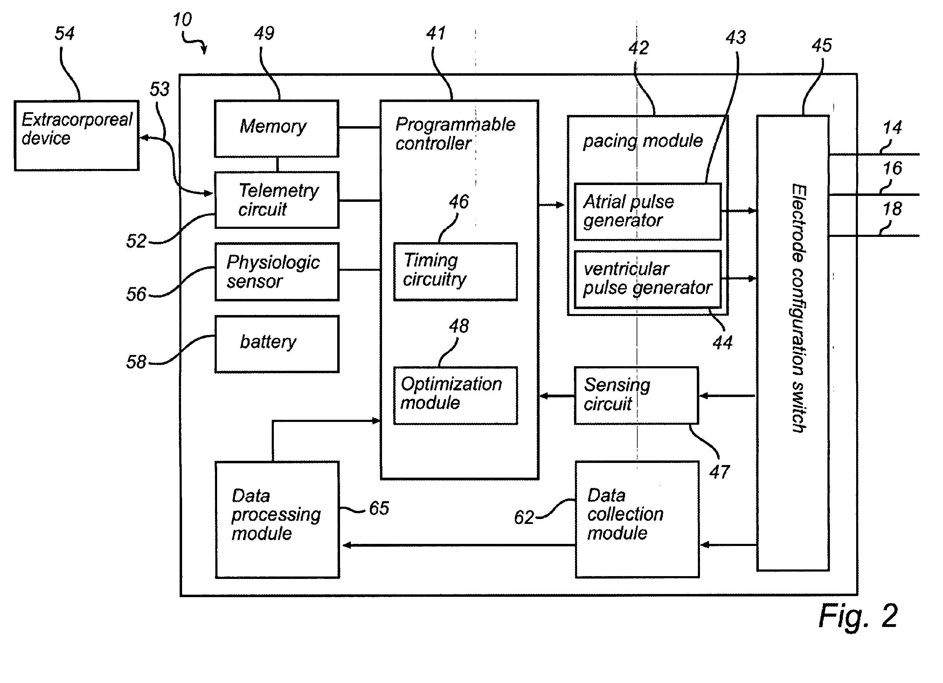

In Fig. 2, an exemplary, simplified block diagram depicting various components of the cardiac stimulator according to embodiments of the present invention is shown. The cardiac stimulator 10 is capable of delivering cardiac resynchronization therapy and is configured to integrate both monitoring and therapy features, as will be described below. The cardiac stimulator 10 collects and processes data about the heart 12 from one or more sensors including at least one hemodynamic sensor 15. In one particular embodiment of the present invention, the hemodynamic sensor is a pressure sensor 15 arranged in a medical lead 13 connectable to the cardiac stimulator 10, see Fig. 1. Further, the cardiac stimulator 10 collects and processes data about the heart 12 from electrode pairs for sensing cardiac electrogram (EGM) signals. While a particular multi-chamber device is shown, it is to be appreciated and understood that this is done for illustration purposes only. Thus, the techniques and methods described below can be implemented in connection with any suitable configured or configurable stimulation device. Accordingly, one of skill in the art could readily duplicate, eliminate, or disable the appropriate circuitry in any desired combination to provide a device capable of treating the appropriate chamber with pacing stimulation including cardiac resynchronisation therapy.

The cardiac stimulator 10 has a housing 40, often referred to as the "can" or "case electrode". The housing 40 may function as a return electrode in "unipolar" modes. Further, the housing 40 includes connector (not shown) having a plurality of terminals (not shown) for connection with electrodes and/or sensors.

The cardiac stimulator 10 includes a programmable microcontroller or control module 41 that inter alia controls the various modes of stimulation therapy. As well known within the art, the microcontroller 41 typically includes a microprocessor, or equivalent control circuitry, designed specifically for controlling the delivery of stimulation therapy and may further include RAM or ROM memory, logic and timing circuitry, state machine circuitry, and I/O circuitry. Typically, the microcontroller 41 includes the ability to process or monitor input signals (data or information) as controlled by a program stored in a designated block of memory. The type of microcontroller is not critical to the described implementations. Rather, any suitable microcontroller 41 may be used that carries out the functions described herein. The use of micro-processor based control circuits for performing timing and data analysis are well known in the art.

Furthermore, the cardiac stimulator 10 includes pacing module 42 adapted to provide pacing signals for delivery to the patient. The pacing module 42 comprises an atrial pulse generator 43 and a ventricular pulse generator 44 that generate pacing stimulation pulses for delivery by the right atrial lead 14, the coronary sinus lead 16, and/or the right ventricular lead 18 via an electrode configuration switch 45. It is understood that in order to provide stimulation therapy in each of the four chambers, the atrial and ventricular pulse generators 43 and 44, may include dedicated, independent pulse generators, multiplexed pulse generators, or shared pulse generators. The pulse generators 43 and 44 are controlled by the microcontroller 41 via appropriate control signals to trigger or inhibit stimulation pulses.

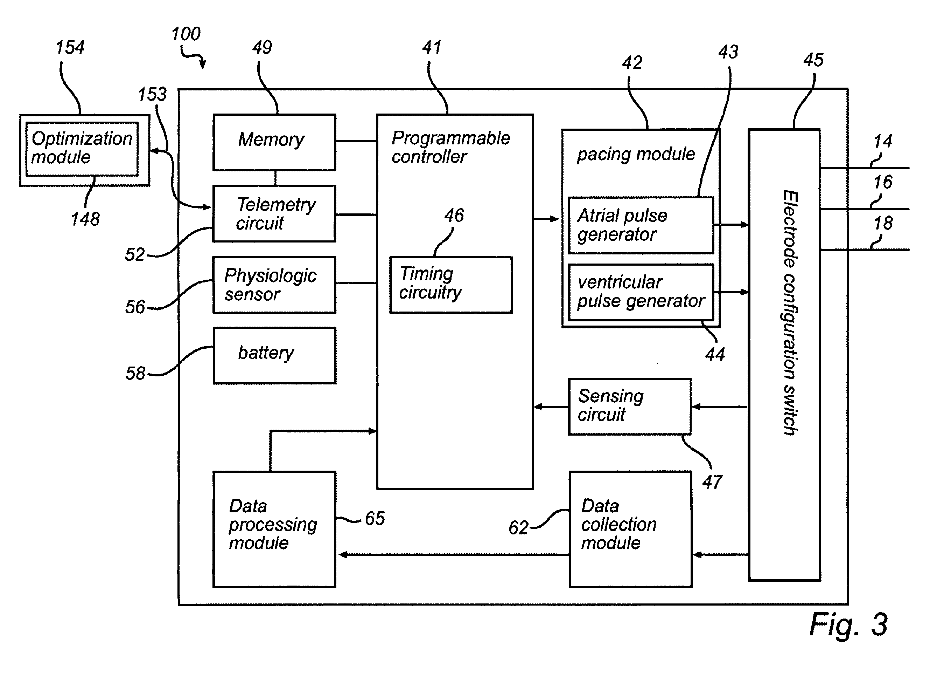

The microcontroller 41 further includes timing control circuitry 46 to control timing of the stimulation pulses (e.g. pacing rate, AV delay, VV delay, etc.) as well as to keep track of timing of refractory periods blanking intervals, etc. which is well known in the art. In addition, the microcontroller 41 may include components such as e.g. an arrhythmia detector (not shown) and/or a morphology detector (not shown). Moreover, an optimization module 48 is adapted to optimize an AV and VV delay based on IEGM signals to determine recommended AV and VV delays. In embodiments of the present invention, the optimization module 48 employs QuickOpt^{™} Timing Cycle Optimization for IEGM based AV timing optimization and VV timing optimization. The aforementioned components may be implemented as part of the microcontroller 41, or as software/firmware instructions programmed into the device and executed on the microcontroller 41 during certain modes of operation. In alternative embodiments the optimization module is arranged in an extracorporeal or external device instead of being arranged in the cardiac stimulator. One example of such an embodiment is shown in Fig. 3. In the embodiment shown in Fig. 3, the cardiac stimulator is denoted by the reference figure 100. The cardiac stimulator 100 has an optimization module 148 arranged in an extracorporeal device 154.

A sensing circuit 47 comprising atrial sensing circuits and ventricular sensing circuits may also be coupled to the right atrial lead 14, the coronary sinus lead 16, and the right ventricular lead 18 through the switch 45 for detecting the presence of cardiac activity in each of the four chambers of the heart. Accordingly, the atrial sensing circuits and ventricular sensing circuits 47 may include dedicated sense amplifiers, multiplexed amplifiers, or shared amplifiers.

The output from the atrial sensing circuits and ventricular sensing circuits 47 are connected to the microcontroller 41, which, in turn, is able to trigger or inhibit the atrial sensing circuits and ventricular sensing circuits 47 in a demand fashion in response to the absence or presence of cardiac activity in the appropriate chamber of the heart.

Furthermore, the microcontroller 41 is coupled to a memory 49 by a suitable data/address bus (not shown), wherein the programmable operating parameters used by the microcontroller 41 are stored and modified, as required, in order to customize the operation of the cardiac stimulator to the needs of a particular patient. Such operating parameters define, for example, pacing pulse amplitude, pulse duration, etc. Advantageously, the operating parameters may be non-invasively programmed into the memory 49 through a communication module 52 including, for example, a telemetry circuit for telemetric communication via communication link 53 with an external device 54, such as a programmer or a diagnostic system analyzer. The telemetry circuit advantageously allows intracardiac electrograms and status information relating to the operation of the device 10 to be sent to the external device 54 through an established communication link 53. Further, the communication module may alternatively or as a complement to the telemetry circuit include circuit for RF communication.

The cardiac stimulator 10 may further include a physiologic sensor 56, commonly referred to as a "rate-responsive" sensor because it is typically used to adjust pacing stimulation rate according to the exercise state of the patient. While shown as being included within the stimulator 10, it is to be understood that the physiologic sensor 56 also may be external to the stimulator, yet still be implanted within or carried by the patient. Examples of physiologic sensors include sensors that, for example, sense respiration rate, or activity variance.

Moreover, the cardiac stimulator 10 additionally includes a battery 58 that provides operating power to all of the circuits shown in Fig. 2. Preferably, the stimulator 10 employs lithium or similar battery technology.

A data collection module 62 is adapted to collect hemodynamic signals from the hemodynamic sensor 15, for example, during different AV delays and VV delays for later processing of the data. The data collection module 62 comprises, for example, analog-to-digital (A/D) data acquisition circuits adapted to acquire and amplify the signals from the hemodynamic sensor 15 and to convert the raw analog data into a digital signal, filter the signals and store the digital signals for later processing in a data processing module 65, which also may be integrated in the microcontroller 41.

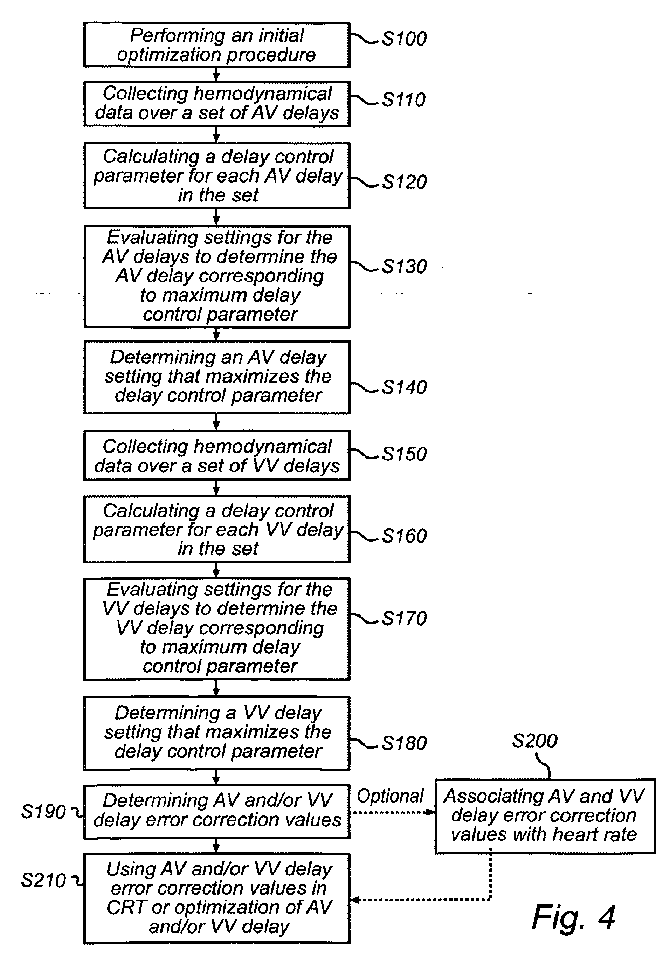

According to embodiments of the present invention, the data processing module 65 is adapted to determine or calculate delay control parameters for AV delays and VV delays based on the collected hemodynamic signals for respective AV delay and VV delay as will be described in more detail below with reference to Fig. 4. These delay control parameters are used to evaluate the settings for the AV delays and VV delays to determine the AV delay setting that corresponds to the maximum delay control parameter and the VV delay setting that corresponds to the maximum delay control parameter. Based on this, an AV delay error correction value is determined as a difference between the AV delay corresponding to the maximum delay control parameter and a recommended AV delay and a VV delay error correction value is determined as a difference between the VV delay corresponding to the maximum delay control parameter and a recommended VV delay. The recommended AV and VV delay are, for example, determined by means of the optimization module 48 employing QuickOpt^{™} Timing Cycle Optimization.

With reference now to Fig. 4, one embodiment of the optimization procedure for obtaining timing data for CRT according to the present invention will be described. First, at step S100, an optimization based on IEGM data is performed to obtain AV and VV timings, for example, the QuickOpt^{™} Timing Cycle Optimization, which is an algorithm that provides IEGM based AV timing optimization in CRT and ICD systems and VV timing optimization in CRT devices. QuickOpt^{™} Timing Cycle Optimization provides recommended AV and VV delays: AVD_{QO} and VVD_{QO}, respectively. Optionally, the heart rate is also stored with a timestamp linking it to the recommended AV and VV delays. The optimization includes providing and delivering pacing signals to the patient via the medical leads 14, 16, 18.

Thereafter, at step S110, a sweep across a preset or predetermined set of AV delays is initiated and the hemodynamic signal (or signals) is simultaneously recorded during each AV delay. In one preferred embodiment, the hemodynamic signal is the left ventricular pressure (LVP). One example of such AV delay (AVD) sweep could be the following: AVD₁ = 60 ms, AVD₂ = 80 ms, AVD₃ = 100 ms, ADV₄ = 120 ms, AVD₅ = 140 ms, AVD₆ = 160 ms, AVD₇ = 180 ms.

For each of these 20 ms delays a sequence of hemodynamic data will be recorded. That is the following hemodynamic data (hd) is recorded: hd₁, hd₂, hd₃, hd₄, hd₅, hd₆, hd₇. In one particular embodiment, the left ventricular pressure is recorded and accordingly: LVP₁, LVP₂, LVP₃, LVP₄, LVP₅, LVP₆, LVP₇.

Subsequently, at step S120, a delay control parameter is calculated for each AV delay. According to one particular embodiment, the left ventricular pressure (LVP) signal is bandpass filtered and differentiated to produce a continous dLVP/dt signal: dLVP₁/dt, dLVP₂/dt, dLVP₃/dt, dLVP₄/dt, dLVP₅/dt, dLVP₆/dt, dLVP₇/dt.

At step S130, the settings for the AV delays are evaluated to determine the AV delay setting that corresponds to the maximum delay control parameter. In one embodiment of the present invention, each sequence of dLVP/dt data will be processed to identify each local maxima of the differentiated LVP signal (i.e. dLVP/dt_{MAX}). In this specific embodiment, the following values are created: dLVP₁/dt_{MAX}, dLVP₂/dt_{MAX}, dLVP₃/dt_{MAX}, dLVP₄/dt_{MAX,} dLVP₅/dt_{MAX}, dLVP₆/dt_{MAX}, dLVP₇/dt_{MAX}.

Thereafter, at step S140, the setting that maximizes the delay control parameter is determined. In the discussed embodiment, the setting that maximizes the differentiated LVP (i.e. max (dLVP/dt_{MAX)}) is determined. According to an embodiment of the present invention, the setting that maximizes the delay control parameter is identified by fitting a 2^{nd} or 3^{rd} degree polynomial to the data points using least squares. The setting that maximizes the delay control parameter is denoted AVp.

At step S150, a second sweep is performed over different (which may be preset or predetermined) VV delays is performed, for example, at AVD = AVp. To counteract drift, a control measurement where VVD is set to zero is performed between each VVD measurement. According to an example, the sweep can be as follows:
VV₁ -80 ms, VV = 0; VV₂ = -60 ms, VV = 0; VV₃ = -40 ms, VV = 0; VV₄ = - 20 ms, VV = 0; VV₅ = 0; VV₆ = 20 ms, VV = 0; VV₇ = 40 ms, VV = 0; VV₈ = 60 ms, VV = 0; VV₁ = 80 ms, VV = 0.

Hemodynamic signal (or signals) is simultaneously recorded during each VV delay. In one preferred embodiment, the hemodynamic signal is the left ventricular pressure (LVP).

At step S160, a delay control parameter is determined for each VV delay. According to one particular embodiment, the left ventricular pressure (LVP) signal is bandpass filtered and differentiated to produce a continous dLVP/dt signal: dLVP₁/dt, dLVP₂/dt, dLVP₃/dt, dLVP₄/dt, dLVP₅/dt, dLVP₆/dt, dLVP₇/dt, dLVP ₈/dt, dLVP₉/dt.

Thereafter, at step S170, the VV delay settings are evaluated to determine the VV delay setting that corresponds to the maximum delay control parameter. In one embodiment of the present invention, each sequence of dLVP/dt data will be processed to identify each local maxima of the differentiated LVP signal (i.e. dLVP/dt_{MAX)}. In this specific embodiment, the following values are created: dLVP₁/dt_{MAX}, dLVP₂/dt_{MAX}, dLVP₃/dt_{MAX}, dLVP₄/dt_{MAX,} dLVP₅/dt_{MAX}, dLVP₆/dt_{MAX}, dLVP₇/dt_{MAX}, dLVP₈/dt_{MAX}, dLVP₉/dt_{MAX}.

At step S180, the setting that maximizes the delay control parameter is determined. In the discussed embodiment, the setting that maximizes the differentiated LVP (i.e. max (dLVP/dt_{MAX)}) is determined. According to an embodiment of the present invention, the setting that maximizes the delay control parameter is identified by fitting a 2^{nd} or 3^{rd} degree polynomial to the data points using least squares. The setting that maximizes the delay control parameter is denoted VVₚ.

Subsequently, at step S190, AV delay error correction values and VV delay error correction values are determined as a difference between the AV and VV delay corresponding to the maximum delay control parameter and a recommended AV delay and VV delay respectively. That is, AV delay error correction values and VV delay error correction values are determined according to:
delta_{AV} = AVD_{QO} - AV_{P}
delta_{VV} = VVD_{QO} - VV_{P}

Then, at step S210, the AV and VV delay error correction values are used in CRT or further optimization for update of the AV and VV delays. Optionally, at step 200, the delta values (delta_{AV} and delta_{VV}) are labeled or associated with an approximate heart rate or heart rate interval during which they were obtained. Accordingly, step S200 is optional and if not used, the algorithm proceeds directly to step S21 0 from step S190.

If the error correction values are associated with or labeled to each AV or VV error correction parameter, it is possible to obtain rate dependent error correction terms and AV and VV delays. The AV and VV delay error correction values may be binned together according to what approximate heart rate or heart rate interval at which they were acquired. For example, if a first procedure to obtain AV and VV delay error correction values were performed between 65 - 75 bpm, and a second procedure to obtain AV and VV delay error correction values were performed between 75 - 85 bpm, the results from these procedures will not be mixed. Instead, this will result in two sets of error correction values, one set of AV and VV delay error correction values of the heart rate interval between 65 - 75 bpm and one set of AV and VV delay error correction values of the heart rate interval between 75 - 85 bpm. Thus, it is possible to obtain heart rate specific error correction values and, in turn, heart rate specific AV and VV delays.

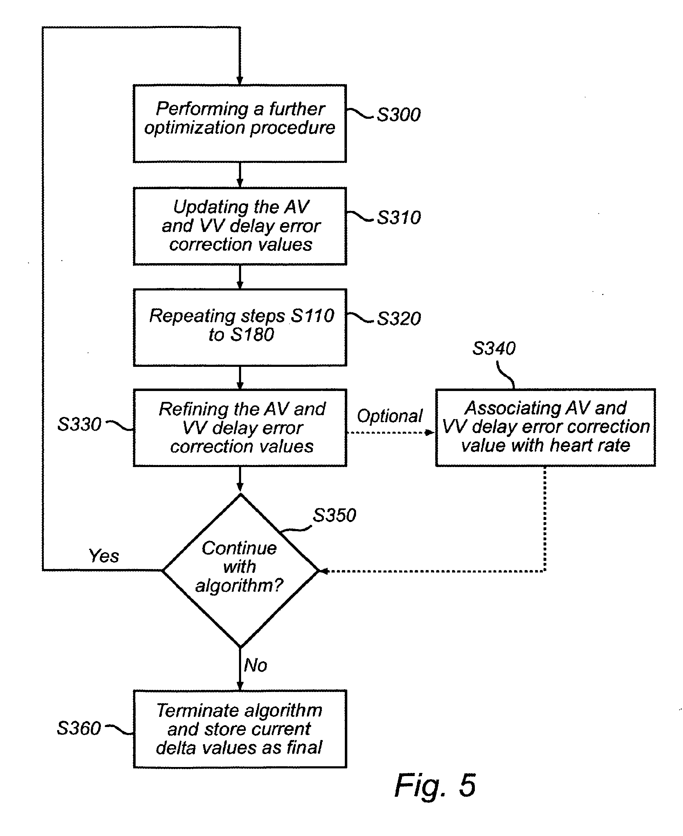

With reference now to Fig. 5, steps performed in block S21 0 according to an embodiment of the present invention will be described.

First, at step S300, a further optimization procedure is performed at a later point of time, for example, a week later than the first optimization procedure. Thus, an optimization based on IEGM data is performed to obtain AV and VV timings, for example, the QuickOpt^{™} Timing Cycle Optimization, QuickOpt^{™} Timing Cycle Optimization provides recommended AV and VV delays: AVD_{QO2} and VVD_{QO2}, respectively. Optionally, the heart rate is also stored with a timestamp linking it to the recommended AV and VV delays. However, the values obtained by means of the optimization procedure is not stored as recommended values but, at step S31 0, these values are updated with the AV and VV delay error correction values, respectively, according to the following:
AVD_{QO-updated} AVD_{QO2} - delta_{AV}
VVD_{QO-updated} AVD_{QO2} - delta_{VV}

Thereafter, at step S320, the steps S110 - S180 are repeated with the updated AV and VV delays as input values.

At step S330, the AV and VV delay error correction values are redefined using the delta_{AV} and delta_{VV} from the latest algorithm loop according to the following:
Delta_{AV}-redefined = delta_{AV_old} + delta_{AV_new}
Delta_{VV_}redefined = delta_{VV_}old + delta_{VV_new}

Accordingly, the delta values from the latest loop are added to the respective delta values from the previous loop. The redefined delta values may optionally be associated or labeled with an approximate heart rate or heart rate interval during which they were obtained at step S340. Accordingly, step S340 is optional and if not used, the algorithm proceeds directly to step S350 from step S330. At step S350, it is checked whether a further optimization should be performed to obtain a new set of updated delta values or if the algorithm should be terminated. If it is determined that a further optimization should be performed, the algorithm returns to step S300. If it is determined that the algorithm should be terminated, the current delta values may be stored as final values for use in the CRT at step S360. For example, if it is verified that the hemodynamic sensor 15 not provides reliable data any longer, e.g. due to over growth, a decision to terminate the algorithm can be made.

Although certain embodiments and examples have been described herein, it will be understood by those skilled in the art that many aspects of the devices shown and described in the present disclosure may be differently combined and/or modified to form still further embodiments. Alternative embodiments and/or uses of the devices described above and obvious modifications and equivalents thereof are intended to be within the scope of the present disclosure. Thus, it is intended that the scope of the present invention should not be limited by the particular embodiments described above, but should be determined by a fair reading of the claims that follow.

## Claims

1. A system (1) comprising a cardiac stimulator (10; 100) for stimulating a heart (12) of a patient, said cardiac stimulator (10; 100) being connectable to a hemodynamic sensor (15) adapted to sense hemodynamic signals and said system (1) further comprising:
a pacing module (42) arranged in said cardiac stimulator (10; 100) adapted to provide pacing signals for delivery to said patient via medical leads (14, 16, 18) including electrodes (20, 21, 22, 23, 24, 28, 30) connectable to said cardiac stimulator (10; 100);
a control module (41) arranged in said cardiac stimulator (10; 100) adapted to control settings of said pacing module (42) to deliver pacing signals in accordance with preset AV and W delays;
a data collection module (62) arranged in said cardiac stimulator (10; 100) adapted to collect hemodynamic signals from said hemodynamic sensor (15) during preset AV delays and W delays;
a data processi module (65) arranged in said cardiac stimulator (10; 100) and being coupable to said data collection module (62); and
an optimization module (48, 148) adapted to optimize an AV and W delay based on IEGM signals obtained via said medical leads (14, 16, 18) to determine recommended AV and W delays; **characterized in that**
said data processing module (65) is adapted to:
determine a delay control parameter for each preset AV delay and W delay based on the collected hemodynamic signals for respective preset AV delay and W delay;
evaluate the settings for said preset AV delays and W delays to determine the AV delay setting that corresponds to the maximum delay control parameter and the W delay setting that corresponds to the maximum delay control parameter;
determine an AV delay error correction value as a difference between the AV delay corresponding to the maximum delay control parameter and a recommended AV delay; and
determine a W delay error correction value as a difference between the W delay corresponding to the maximum delay control parameter and a recommended W delay.

2. The system according to claim 1, wherein said data processing module (65) is adapted to:
update said recommended AV and/or W delay with said AV and/or W delay error correction value, respectively, to obtain a modified recommended AV and/or W delay.

3. The system according to claim 1 or 2, wherein said data processing module (65) is adapted to:
modify current AV and W delay error correction values using at least one set of AV and W delay error correction values obtained from a subsequent determination of AV and W delay error correction values.

4. The system according to any one of claims 1 - 3, wherein
said control module (41) is adapted to control said pacing module (42) to deliver pacing signals in accordance with preset W delays at a specific AV delay, said specific AV delay being the AV delay corresponding to the maximum delay control parameter; and wherein
said data processing module (65) is adapted to:
determine a delay control parameter for each VV delay based on the collected hemodynamic signals for respective W delay obtained at said specific AV delay; and
evaluate the settings for the W delays to determine the W delay setting that corresponds to the maximum delay control parameter at said specific AV delay.

5. The system according to any one of claims 1 - 4, wherein said hemodynamic sensor (15) is an implantable pressure sensor.

6. The system according to claim 5, wherein said pressure sensor is adapted for transmural placement so as to enable sensing of a left ventricular pressure.

7. The system according to any one of claims 1 - 4, wherein said hemodynamic sensor (15) is selected from the group including: accelerometers, blood flow probes, load indicators which react to geometrical changes, heart sound sensors, and photoplethysmography sensors.

8. The system according to any one of claims 1 - 7, wherein said cardiac stimulator (10; 100) further comprises a communication module (52) adapted to communicate (53) with extracorporeal equipment (54) wirelessly using RF or inductive telemetry.

9. The system according to claim 8, wherein said optimization module (148) is arranged in extracorporeal equipment (154) having a communication module for communication with said communication module (52) of said cardiac stimulator (100).

10. The system according to any one of claims 1 - 8, wherein said optimization module (48) is arranged in said cardiac stimulator (10).

11. The system according to claim 5, 6, and 8 - 10 when depending on claim 5 or 6, where in said data processing module (65) is adapted to calculate the left ventricular pessure derivative for each AV and VV delay, wherein said delay control parameter is said left ventricular pressure derivative.

12. The system according to any one of preceding claims, wherein said data processing module (65) is adapted to determine a heart rate or heart rate interval based on the sensed heart rate at which the hemodynamic signals were obtained.

13. The system according to claim 12, wherein said data processing module (65) is adapted to associate a heart rate or heart rate interval with each AV and/or VV error correction parameter.

## Patentansprüche

1. System (1), umfassend einen Herzstimulator (10; 100) zum Stimulieren eines Herzens (12) eines Patienten, wobei der Herzstimulator (10; 100) mit einem hämodynamischen Sensor (15) verbindbar ist, der so ausgelegt ist, dass er hämodynamische Signale erfasst, und wobei das System (1) ferner Folgendes umfasst:
ein in dem Herzstimulator (10; 100) angeordnetes Stimulationsmodul (42), das so ausgelegt ist, dass es Stimulationssignale zum Abgeben an den Patienten über medizinische Leitungen (14, 16, 18) liefert, die Elektroden (20, 21, 22, 23, 24, 28, 30) aufweisen, die mit dem Herzstimulator (10; 100) verbindbar sind;
ein in dem Herzstimulator (10; 100) angeordnetes Steuermodul (41), das so ausgelegt ist, dass es Steuereinstellungen des Stimulationsmoduls (42) steuert, damit es Stimulationssignale gemäß voreingestellter AV- und VV-Intervalle abgibt;
ein in dem Herzstimulator (10; 100) angeordnetes Datenerfassungsmodul (62), das so ausgelegt ist, das es von dem hämodynamischen Sensor (15) während voreingestellter AV-Intervalle und VV-Intervalle hämodynamische Signale erfasst;
ein Datenverarbeitungsmodul (65), das in dem Herzstimulator (10; 100) angeordnet und mit dem Datenerfassungsmodul (62) verbindbar ist; und
ein Optimierungsmodul (48, 148), das so ausgelegt ist, dass es ein AV- und VV-Intervall auf der Grundlage von IEGM-Signalen optimiert, die über die medizinischen Leitungen (14, 16, 18) erhalten werden, um empfohlene AV- und VV-Intervalle zu ermitteln; **dadurch gekennzeichnet, dass**
das Datenverarbeitungsmodul (65) so ausgelegt ist, dass es:
einen Intervall-Steuerparameter für jedes voreingestellte AV-Intervall und W-Intervall auf der Grundlage der erfassten hämodynamischen Signale für das jeweilige voreingestellte AV-Intervall und VV-Intervall ermittelt;
die Einstellungen für die voreingestellten AV-Intervalle und W-Intervalle beurteilt, um die AV-Intervall-Einstellung, die dem maximalen Intervall-Steuerparameter entspricht, und die VV-Intervall-Einstellung zu ermitteln, die dem maximalen Intervall-Steuerparameter entspricht;
einen AV-Intervall-Fehlerkorrekturwert als Unterschied zwischen dem AV-Intervall, das dem maximalen Intervall-Steuerparameter entspricht, und einem empfohlenen AV-Intervall ermittelt; und
einen VV-Intervall-Fehlerkorrekturwert als Unterschied zwischen dem VV-Intervall, das dem maximalen Intervall-Steuerparameter entspricht, und einem empfohlenen W-Intervall ermittelt.

2. System nach Anspruch 1, wobei das Datenverarbeitungsmodul (65) so ausgelegt ist, dass es:
das empfohlene AV- und/oder W-Intervall mit dem AV- beziehungsweise VV-Intervall-Fehlerkorrekturwert aktualisiert, um ein modifiziertes empfohlenes AV- und/oder W-Intervall zu erhalten.

3. System nach Anspruch 1 oder 2, wobei das Datenverarbeitungsmodul (65) so ausgelegt ist, dass es:
aktuelle AV- und VV-Intervall-Fehlerkorrekturwerte unter Verwendung von mindestens einem Satz von AV- und VV-Intervall-Fehlerkorrekturwerten, die aus einer späteren Ermittlung von AV- und VV-Intervall-Fehlerkorrekturwerten erhalten werden, modifiziert.

4. System nach einem der Ansprüche 1 bis 3, wobei
das Steuermodul (41) so ausgelegt ist, dass es das Stimulationsmodul (42) steuert, damit es Stimulationssignale gemäß voreingestellter VV-Intervalle bei einem bestimmten AV-Intervall abgibt, wobei das bestimmte AV-Intervall das AV-Intervall ist, das dem maximalen Intervall-Steuerparameter entspricht; und wobei
das Datenverarbeitungsmodul (65) so ausgelegt ist, dass es:
einen Intervall-Steuerparameter für jedes W-Intervall auf der Grundlage der erfassten hämodynamischen Signale für das jeweilige W-Intervall ermittelt, die bei dem bestimmten AV-Intervall erhalten werden; und
die Einstellungen für die VV-Intervalle beurteilt, um die VV-Intervall-Einstellung, die dem maximalen Intervall-Steuerparameter bei dem bestimmten AV-Intervall entspricht, zu ermitteln.

5. System nach einem der Ansprüche 1 bis 4, wobei der hämodynamische Sensor (15) ein implantierbarer Drucksensor ist.

6. System nach Anspruch 5, wobei der Drucksensor zur transmuralen Platzierung ausgelegt ist, um die Erfassung eines linksventrikulären Drucks zu ermöglichen.

7. System nach einem der Ansprüche 1 bis 4, wobei der hämodynamische Sensor (15) aus der Gruppe ausgewählt ist, die Folgendes umfasst: Beschleunigungsmesser, Blutflusssensoren, Belastungsanzeiger, die auf geometrische Veränderungen reagieren, Herztonsensoren und Photoplethysmographie-Sensoren.

8. System nach einem der Ansprüche 1 bis 7, wobei der Herzstimulator (10; 100) ferner ein Datenübertragungsmodul (52) umfasst, das so ausgelegt ist, dass es mit Einrichtungen außerhalb des Körpers (54) drahtlos unter Verwendung von RF- oder induktiver Telemetrie, Daten austauschen (53) kann.

9. System nach Anspruch 8, wobei das Optimierungsmodul (148) in Einrichtungen außerhalb des Körpers (154) mit einem Datenübertragungsmodul für den Datenaustausch mit dem Datenübertragungsmodul (52) des Herzstimulators (100) angeordnet ist.

10. System nach einem der Ansprüche 1 bis 8, wobei das Optimierungsmodul (48) in dem Herzstimulator (10) angeordnet ist.

11. System nach Anspruch 5, 6 und 8 bis 10, wenn von Anspruch 5 oder 6 abhängig, wobei das Datenverarbeitungsmodul (65) so ausgelegt ist, dass es die Ableitung des linksventrikulären Drucks für jedes AV- und W-Intervall berechnet, wobei der Intervall-Steuerparameter die Ableitung des linksventrikulären Drucks ist.

12. System nach einem der vorhergehenden Ansprüche, wobei das Datenverarbeitungsmodul (65) so ausgelegt ist, dass es eine Herzfrequenz oder ein RR-Intervall auf der Grundlage der erfassten Herzfrequenz, bei der die hämodynamischen Signale erhalten wurden, ermittelt.

13. System nach Anspruch 12, wobei das Datenverarbeitungsmodul (65) so ausgelegt ist, dass es eine Herzfrequenz oder ein RR-Intervall mit jedem AV- und/oder VV-Fehlerkorrekturparameter verknüpft.

## Revendications

1. Système (1) comprenant un stimulateur cardiaque (10 ; 100) pour stimuler un coeur (12) d'un patient, ledit stimulateur cardiaque (10 ; 100) pouvant être raccordé à un capteur hémodynamique (15) adapté pour détecter des signaux hémodynamiques et ledit système (1) comprenant en outre :
un module de stimulation (42) agencé dans ledit stimulateur cardiaque (10 ; 100) adapté pour fournir des signaux de stimulation pour les délivrer audit patient via des dérivations médicales (14, 16, 18) comportant des électrodes (20, 21, 22, 23, 24, 28, 30) pouvant être raccordées audit stimulateur cardiaque (10 ; 100) ;
un module de commande (41) agencé dans ledit stimulateur cardiaque (10 ; 100) adapté pour commander des réglages dudit module de stimulation (42) pour délivrer des signaux de stimulation conformément à des délais AV et W prérèglés ;
un module de collecte de données (62) agencé dans ledit stimulateur cardiaque (10 ; 100) adapté pour recueillir des signaux hémodynamiques dudit capteur hémodynamique (15) pendant des délais AV et des délais VV préréglés ;
un module de traitement de données (65) agencé dans ledit stimulateur cardiaque (10 ; 100) et pouvant être couplé audit module de collecte de données (62) ; et
un module d'optimisation (48, 148) adapté pour optimiser un délai AV et VV sur la base de signaux d'électrogramme endocavitaire obtenus via lesdites dérivations médicales (14, 16, 18) pour déterminer des délais AV et VV recommandés ; **caractérisé en ce que**
ledit module de traitement de données (65) est adapté pour :
déterminer un paramètre de commande de délai pour chaque délai AV et délai VV préréglé sur la base des signaux hémodynamiques recueillis pour le délai AV et le délai VV préréglés respectifs ;
évaluer les réglages pour lesdits délais AV et délais VV préréglés pour déterminer le réglage de délai AV qui correspond au paramètre de commande de délai maximal et le réglage de délai VV qui correspond au paramètre de commande de délai maximal ;
déterminer une valeur de correction d'erreur de délai AV en tant que différence entre le délai AV correspondant au paramètre de commande de délai maximal et un délai AV recommandé ; et
déterminer une valeur de correction d'erreur de délai VV en tant que différence entre le délai VV correspondant au paramètre de commande de délai maximal et un délai VV recommandé.

2. Système selon la revendication 1, dans lequel ledit module de traitement de données (65) est adapté pour :
mettre à jour ledit délai AV et/ou VV recommandé avec respectivement ladite valeur de correction d'erreur de délai AV et/ou VV, pour obtenir un délai AV et/ou VV recommandé modifié.

3. Système selon la revendication 1 ou 2, dans lequel ledit module de traitement de données (65) est adapté pour :
modifier des valeurs actuelles de correction d'erreur de délais AV et VV en utilisant au moins un ensemble de valeurs de correction d'erreur de délais AV et VV obtenu d'une détermination subséquente de valeurs de correction d'erreur de délais AV et VV.

4. Système selon l'une quelconque des revendications 1 à 3, dans lequel
ledit module de commande (41) est adapté pour commander ledit module de stimulation (42) pour délivrer des signaux de stimulation conformément à des délais VV préréglés à un délai AV spécifique, ledit délai AV spécifique étant le délai AV correspondant au paramètre de commande de délai maximal ; et dans lequel
ledit module de traitement de données (65) est adapté pour :
déterminer un paramètre de commande de délai pour chaque délai VV sur la base des signaux hémodynamiques recueillis pour le délai VV respectif obtenu audit délai AV spécifique ; et
évaluer les réglages pour les délais VV pour déterminer le réglage de délai VV qui correspond au paramètre de commande de délai maximal audit délai AV spécifique.

5. Système selon l'une quelconque des revendications 1 à 4, dans lequel ledit capteur hémodynamique (15) est un capteur de pression implantable.

6. Système selon la revendication 5, dans lequel ledit capteur de pression est adapté pour un placement transmural de façon à permettre la détection d'une pression ventriculaire gauche.

7. Système selon l'une quelconque des revendications 1 à 4, dans lequel ledit capteur hémodynamique (15) est sélectionné dans le groupe comprenant : accéléromètres, transducteurs de débit sanguin, indicateurs de charge qui réagissent aux changements géométriques, capteurs de bruits cardiaques, et capteurs de photopléthysmographie.

8. Système selon l'une quelconque des revendications 1 à 7, dans lequel ledit stimulateur cardiaque (10 ; 100) comprend en outre un module de communication (52) adapté pour communiquer (53) avec des équipements extracorporels (54) sans fil en utilisant la télémétrie RF ou inductive.

9. Système selon la revendication 8, dans lequel ledit module d'optimisation (148) est agencé dans des équipements extracorporels (154) présentant un module de communication pour la communication avec ledit module de communication (52) dudit stimulateur cardiaque (100).

10. Système selon l'une quelconque des revendications 1 à 8, dans lequel ledit module d'optimisation (48) est agencé dans ledit stimulateur cardiaque (10).

11. Système selon les revendications 5, 6 et 8 à 10 quand elles dépendent de la revendication 5 ou 6, dans lequel ledit module de traitement de données (65) est adapté pour calculer la dérivée de la pression ventriculaire gauche pour chaque délai AV et VV, ledit paramètre de commande de délai étant ladite dérivée de la pression ventriculaire gauche.

12. Système selon l'une quelconque des revendications précédentes, dans lequel ledit module de traitement de données (65) est adapté pour déterminer une fréquence cardiaque ou un intervalle de fréquence cardiaque sur la base de la fréquence cardiaque détectée à laquelle les signaux hémodynamiques ont été obtenus.

13. Système selon la revendication 12, dans lequel ledit module de traitement de données (65) est adapté pour associer une fréquence cardiaque ou un intervalle de fréquence cardiaque à chaque paramètre de correction d'erreur AV et/ou VV.
